# DEMANDE DE BREVET EUROPEEN

(11) **EP 0 624 345 A1**
(43) Date de publication de la demande: **17.11.1994**
(21) Numéro de dépôt: 94400873.9
(22) Date de dépôt: 22.04.1994
(51) Int. Cl.: A61B 17/32, B23D 33/08, B23D 29/02

(54) **Pince coupante stérile pour un fil plus ou moins rigide**

(30) Priorité: 14.05.1993 FR 9305829
(71) Demandeur: Bourrelly, Bernard J., F-77150 Lesigny (FR)
(72) Inventeur: Bourrelly, Bernard J., F-77150 Lesigny (FR)
(74) Mandataire: Dronne, Guy

(57) **Abrégé**

L'invention concerne une pince coupante stérile destinée à sectionner un fil plus ou moins rigide (8).

Cette pince coupante comporte un premier levier (10) et un second levier (12) réunis par un axe (14). Chaque levier présente une arête de découpage (20, 22).

Suivant l'invention, la pince coupante comporte en outre un organe (24) de blocage du fil (8), monté pivotant sur l'axe (14) ou sur un axe solidaire de l'un des leviers et présentant une face active (36).

Pendant et après la découpe, le fil (8) ou son extrémité coupée (34) est maintenu bloqué entre ladite face active (36) et la première arête de découpage (20).

## Description

La présente invention concerne une pince coupante stérile destinée à sectionner un fil plus ou moins rigide.

Par fil plus ou moins rigide, on entend par exemple la tige d'une brosse de prélèvement microbiologique protégé. Une telle brosse est utilisée dans une sonde de prélèvement, introduite à l'aide d'un cathéter dans le corps humain, par exemple dans les poumons, pour effectuer des prélèvements qui feront ensuite l'objet d'une analyse bactériologique.

On comprendra donc que la tige d'une telle sonde doit être suffisamment souple pour suivre sans dommage les méandres du corps humain et suffisamment rigide pour être guidable dans la sonde.

Le fil plus ou moins rigide auquel s'applique l'invention peut donc être constitué de fils métalliques fins torsadés, d'un fil nylon, plastique ou autre.

L'analyse bactériologique est réalisée à partir de la brosse de prélèvement dont la tige doit être sectionnée et qui doit être recueillie dans un réceptacle d'analyse.

Les résultats de l'analyse bactériologique ne sont fiables que si l'ensemble de l'opération, depuis l'introduction de la sonde jusqu'au sectionnement de la tige de la brosse, est réalisée en milieu stérile.

Les pinces coupantes connues comportent deux leviers réunis par un axe qui sépare chacun d'entre eux en un bras court et en un bras long. Le bras court de chacun des deux leviers présente, près de son extrémité, une arête de découpage. Les deux leviers fonctionnent à la manière d'une paire de ciseaux en pivotant l'un sur l'autre de manière à rapprocher les deux arêtes de découpage placées en vis-à-vis. Ainsi un fil rigide, ou la tige d'une brosse de prélèvement microbiologique, peut être sectionné.

D'autres pinces connues comportent deux leviers qui sont réunis par un axe à une première extrémité. Chaque levier comporte une arête de découpage placée entre l'axe et une seconde extrémité. Les leviers sont susceptibles de pivoter l'un par rapport à l'autre en ramenant l'une vers l'autre les deux arêtes de découpage.

Avec de telles pinces connues, le mouvement de cisaillement des deux arêtes de découpage projette la partie d'extrémité coupée comportant la brosse de prélèvement dans une direction aléatoire. Ceci est particulièrement dommageable dans la mesure où la brosse de prélèvement doit être recueillie dans un réceptacle stérile en vue de procéder à l'analyse bactériologique et où il est indispensable d'éviter qu'elle entre en contact avec un élément non stérile avant cette analyse.

Le maniement des pinces connues est donc délicat puisque le praticien doit maintenir la partie à couper au-dessus de l'ouverture d'un réceptacle stérile tout en coupant le fil rigide servant de tige à la brosse et en s'assurant que celle-ci soit effectivement recueillie dans le réceptacle.

De plus, de telles pinces sont généralement relativement coûteuses et, dans la mesure où l'opération doit être réalisée en milieu stérile, elles doivent être stérilisées avant chaque usage, ce qui est à la fois fastidieux et coûteux en énergie.

On connaît aussi, par le brevet US 1 444 044, une pince coupante munie d'un organe de blocage du fil à découper. Cet organe est fixé aux leviers de la pince par des branches formant ressort.

La fabrication d'une telle pince est relativement coûteuse. De plus, l'encombrement de cette pince est important, ce qui représente un inconvénient lorsque l'on sait que la longueur de la zone dans laquelle on peut couper la tige d'une brosse de prélèvement microbiologique est limitée d'un côté par l'extrémité de la gaine du cathéter et, de l'autre, par la brosse elle-même.

La présente invention a pour but de remédier aux inconvénients des pinces connues en proposant une pince coupante du type précité, munie d'un organe de blocage du fil, de maniement simple, permettant de maintenir le fil avant sa découpe et de retenir la partie d'extrémité coupée avant de la déposer dans un réceptacle d'analyse.

La présente invention propose donc une pince coupante stérile, destinée à sectionner un fil plus ou moins rigide et à le maintenir pendant et après sa découpe, cette pince coupante étant susceptible d'être manoeuvrée d'une seule main, indifféremment droite ou gauche.

La présente invention a également pour objet de proposer une pince coupante stérile d'un coût modeste et donc susceptible d'être jetée après emploi.

Un but supplémentaire de la présente invention est de proposer une pince coupante stérile, livrée dans le même emballage stérile que la sonde de prélèvement bactériologique afin d'en faciliter l'emploi.

La pince coupante objet de la présente invention comporte donc un organe de blocage du fil monté pivotant sur l'axe de pivotement des deux leviers et monté adjacent à la face extérieure de l'un d'entre eux. Les deux leviers jouant des rôles symétriques, on adoptera dans la suite la convention suivant laquelle le levier adjacent à l'organe de blocage est dit premier levier, l'autre levier étant appelé second levier. Suivant cette convention, la première arête de découpage est celle qui est formée sur le bras court du premier levier et la seconde arête de découpage désigne celle qui est formée sur le bras court du second levier.

Pour simplifier la description, on parlera de face intérieure de l'un des leviers pour qualifier la face tournée vers l'autre et de face intérieure de l'organe de blocage pour qualifier la face de cet organe qui est tournée vers le levier adjacent. Les faces extérieures sont évidemment les faces opposées aux faces intérieures.

L'organe de blocage du fil présente une encoche s'étendant à partir de sa périphérie vers sa partie centrale et dont la dimension est adaptée à la section du fil à sectionner.

En vue du découpage, l'organe de blocage du fil est susceptible de venir par pivotement dans une position où le fil peut être inséré dans l'encoche précédemment décrite et entre les deux arêtes de découpage. La partie extrême à couper du fil s'étend alors vers l'extérieur du premier levier, au-delà de la face extérieure de l'organe de blocage.

Selon l'invention, la pince comporte un contact de frottement apte, lors du rapprochement des première et seconde arêtes de découpage, à entraîner l'organe de blocage en pivotement avec le second levier, ledit organe de blocage étant ainsi susceptible d'être sollicité par frottement lors du découpage, de manière à ramener une face active constituée par une face de l'encoche placée vis-à-vis de la première arête de découpage, vers ladite première arête de découpage, de telle sorte que l'extrémité de la partie extrême coupée du fil partiellement engagée dans l'encoche soit maintenue bloquée entre ladite face active et ladite première arête de découpage.

Grâce à cette conformation, la pince coupante peut être réalisée de manière simple et pour un coût modeste. De plus, l'organe de blocage peut présenter de faibles dimensions et l'encombrement de la pince s'en trouve limité. La présence d'un tel contact de frottement permet un fonctionnement automatique de l'organe de blocage puisque la face active de l'encoche de ce dernier est automatiquement entraînée vers la première arête de découpage lors d'un pivotement des deux leviers ramenant les deux arêtes de découpage l'une vers l'autre.

La position de blocage est maintenue jusqu'à ce que, par une sollicitation inverse de l'organe de blocage, la partie extrême du fil soit relâchée pour être déposée à l'endroit voulu, par exemple dans le réceptacle d'analyses.

De manière avantageuse, cette sollicitation inverse est obtenue grâce au contact de frottement qui est apte, lors de l'écartement des arêtes de découpage, à entraîner l'organe de blocage en pivotement avec le second levier.

Une alternative consiste à doter l'organe de blocage d'une zone de manipulation permettant de le solliciter manuellement pour écarter la face active de cet organe de blocage de la première arête de découpage.

Pour ce faire, l'organe de blocage peut, par exemple, être réalisé sous la forme d'un levier ou d'une molette (c'est-à-dire que la zone de manipulation est constituée par un prolongement ou par une denture de sa périphérie externe), de telle sorte qu'il soit susceptible d'être actionné manuellement, par exemple à l'aide du pouce de l'utilisateur.

Dans ce cas, le contact de frottement est mis à contribution pour entraîner l'organe de blocage lors du découpage, mais c'est la sollicitation manuelle qui dégage la partie extrême coupée du fil. Il n'est ainsi plus nécessaire d'ouvrir la pince pour relâcher cette partie extrême coupée.

Selon un mode préféré de réalisation de l'invention, l'axe de pivotement comporte un épaulement annulaire de butée qui le sépare en deux parties et sert d'un côté de butée axiale à l'organe de blocage et de l'autre côté, de butée axiale au levier adjacent, c'est-à-dire au premier levier. L'organe de blocage présente un alésage de butée qui reçoit l'axe et dont l'alésage le plus grand a un diamètre sensiblement égal au diamètre de l'épaulement annulaire de butée et une profondeur adaptée à l'épaisseur de cet épaulement annulaire de butée. Ainsi, l'organe de blocage étant placé sur la première partie de l'axe avec l'alésage le plus grand adjacent à l'épaulement annulaire de butée, celui-ci est coiffé par l'alésage le plus grand sur une épaisseur prédéterminée. Les deux leviers sont placés juxtaposés sur la seconde partie de l'axe, qui constitue leur axe d'articulation.

Ce mode de réalisation permet au premier levier et à l'organe de blocage de pivoter indépendamment l'un de l'autre jusqu'à la position de blocage du fil à découper.

Selon un mode préféré de réalisation, le second levier est rendu solidaire de l'axe en rotation, tandis que le premier levier est monté en pivotement libre autour de cet axe.

Dans ce mode de réalisation, le contact de frottement est réalisé par la coopération par frottement de l'épaulement annulaire de butée avec l'organe de blocage, par l'intermédiaire d'au moins une face (la face périphérique ou le fond) de l'alésage le plus grand de l'organe de blocage. Ce contact par frottement assure une solidarisation partielle en rotation de l'organe de blocage avec l'axe. Plus précisément, lorsque le deuxième levier est actionné en pivotement pour ramener la deuxième arête de découpage vers la première arête de découpage, l'organe de blocage est entraîné vers la première arête de découpage jusqu'à ce que la face active de l'encoche vienne au contact du fil rigide à sectionner et repousse ce dernier en position bloquée entre cette face active de l'encoche et la première arête de découpage.

Selon un autre mode de réalisation, l'organe de blocage comporte, sur sa face intérieure, une saillie formée dans une région qui se trouve en regard de la face intérieure du deuxième levier au moins sur une partie de la course de ce dernier. Cette saillie s'étend parallèlement à l'axe de telle sorte que sa face intérieure constitue une surface de frottement qui, en coopérant avec la zone en regard de la face intérieure du second levier, réalise le contact de frottement.

Les deux exemples de réalisation du contact de frottement qui viennent d'être évoqués peuvent être réalisés indépendamment l'un de l'autre ou être associés.

De préférence, la saillie de la face intérieure de l'organe de blocage est formée le long de la face active de l'encoche. Cette dernière comporte alors une partie qui est située au même niveau que la première arête de découpage. La présence d'une telle saillie permet d'améliorer le blocage du fil qui est retenu sensiblement parallèlement à l'axe. Sans cette saillie, le fil est également bloqué mais est retenu en biais par rapport à l'axe. Le blocage de la partie d'extrémité coupée du fil parallèlement à l'axe permet de positionner aisément cette partie coupée dans l'ouverture d'un réceptacle d'analyse avant de la relâcher dans ce dernier.

Selon une autre variante de réalisation de l'invention, l'organe de blocage peut être monté en pivotement sur un axe décalé par rapport à l'axe d'articulation des leviers et parallèle à ce dernier. Dans ce cas, l'organe de blocage présente une face active susceptible d'être amenée vers la première arête de découpage et de maintenir le fil à couper en position bloquée contre ladite arête de découpage. Le décalage des axes de pivotement améliore la manoeuvrabilité de l'organe de blocage qui est plus accessible.

L'axe de pivotement de l'organe de blocage peut par exemple être placé sur le bras long du premier levier, auquel cas la face intérieure de l'organe de blocage est adjacente à la face extérieure de ce premier levier. L'organe de blocage ainsi positionné peut par exemple être manoeuvré par le pouce pour écarter sa face active de la première arête de découpage.

L'axe de pivotement de l'organe de blocage peut également être placé sur le bras court du second levier, cet organe étant alors de préférence placé adjacent à la face intérieure du bras court du second levier et comporte avantageusement un prolongement qui s'étend à l'extérieur du second levier et sur lequel on peut agir manuellement pour libérer la partie extrême coupée du fil.

Dans cette variante, et lorsque la face intérieure de l'organe de blocage est montée adjacente à la face extérieure du premier levier, le contact de frottement peut être réalisé à l'aide de la face intérieure d'une saillie telle que précédemment évoquée.

L'organe de blocage présente avantageusement une butée destinée à coopérer avec une face de butée du premier levier en position d'ouverture de la pince. Cette butée est située près de la périphérie de l'organe de blocage et est écartée de la face active de ce dernier (éventuellement la face active de l'encoche) de telle façon, que lorsqu'elle coopère avec la face de butée du premier levier, ladite face active soit située entre les deux arêtes de découpage et suffisamment écartée de la première arête de découpage pour permettre l'insertion du fil rigide à sectionner. Grâce à cette butée, le seul geste d'ouverture des deux leviers écarte les deux arêtes de découpage l'une de l'autre et positionne la face active de l'organe de blocage de telle façon que le fil à sectionner peut être introduit à la fois entre les deux arêtes de découpage et entre la face active de l'organe de blocage et la première arête de découpage.

Une telle butée combinée au contact de frottement précédemment décrit rend le maniement de la pince particulièrement aisé.

Selon une autre variante, la pince coupante stérile comprend deux leviers qui sont reliés, par leur première extrémité, à un même axe. Dans ce cas, les première et seconde arêtes de découpage des premier et second leviers sont placées entre l'axe et la seconde extrémité de chaque levier. De même que dans les variantes précédemment décrites, le découpage est réalisé en rapprochant les deux faces de découpage l'une de l'autre, par pivotement des leviers. L'organe de blocage est alors monté pivotant sur l'axe d'extrémité des leviers et présente une face active telle que précédemment décrite.

L'axe peut comporter un épaulement de butée, coiffé par un alésage étagé de l'organe de blocage qui peut être monté avec un contact de frottement avec l'axe ou avec le second levier, comme il a été précédemment décrit. Cet organe de blocage peut également comporter une butée servant à maintenir sa face active écartée de la première arête de découpage en position d'ouverture des leviers.

Dans l'un quelconque des modes de réalisation, les deux leviers sont avantageusement identiques l'un à l'autre afin que la pince coupante puisse indifféremment être utilisée par des gauchers ou des droitiers. Toutefois, lorsque l'organe de blocage est sollicité manuellement pour dégager la partie extrême coupée du fil, on peut prévoir des pinces différentes pour droitiers et pour gauchers dans lesquelles l'organe de blocage est placé d'un côté ou de l'autre des leviers, pour permettre un mouvement naturel de manipulation. Ces leviers sont par exemple obtenus par découpe dans une tôle d'acier de faible épaisseur et dont la résistance est comprise entre 60 et 70 kg/mm². Les axes peuvent être simplement réalisés par décolletage, tandis que l'organe de blocage est en plastique moulé ou en métal embouti. Les coûts de fabrication de la pince sont donc modiques, ce qui permet de l'utiliser une seule fois avant de la jeter. Dans la mesure où la sonde de prélèvement microbiologique protégé est elle-même à usage unique, il est évidemment avantageux de livrer cette sonde et la pince dans le même emballage stérile.

L'invention sera bien comprise et ses avantages apparaîtront mieux à la lecture de la description qui suit, donnée à titre d'exemple non limitatif. La description se réfère aux figures sur lesquelles :
la figure 1 est une vue de côté d'une première variante de la pince coupante,
la figure 2 représente la pince coupante de la figure 1 suivant la flèche F avant le sectionnement du fil,
la figure 3 est la même vue que la figure 2 après le sectionnement du fil,
la figure 4 représente vue de côté la pince coupante dont le deuxième levier est suggéré par des pointillés, en position d'ouverture,
la figure 5 est la même vue que la figure 4 en position de blocage du fil,
la figure 6 est une vue en perspective des divers éléments de la pince avant montage,
la figure 7 représente la pince coupante avec un organe selon un autre mode de réalisation,
la figure 8 représente un organe de blocage selon un autre mode de réalisation,
la figure 9 représente la pince coupante selon une autre variante,
la figure 10 représente la pince coupante selon encore une autre variante.

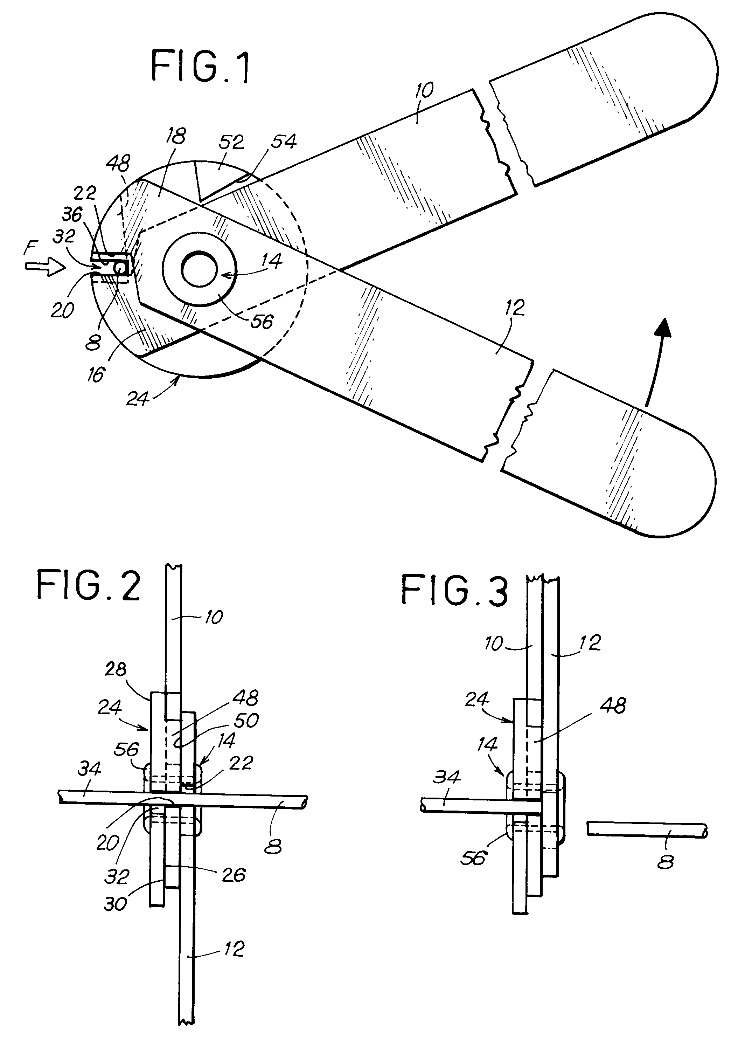

Par référence aux figures 1 à 5, on décrit une pince coupante stérile destinée à sectionner un fil rigide 8 et à le maintenir pendant et après sa découpe. Cette pince comprend un premier levier 10 et un second levier 12 réunis par un axe 14 qui sépare chaque levier en un bras long et un bras court. Le bras court 16 du premier levier présente, près de son extrémité, une première arête de découpage 20. De même, le bras court 18 du second levier présente une seconde arête de découpage 22. Les leviers sont montés de telle façon que les deux arêtes de découpage, 20 et 22, sont placées en vis-à-vis.

Les leviers sont destinés à pivoter l'un par rapport à l'autre dans le sens indiqué, en rapprochant les arêtes de découpage l'une de l'autre. Ainsi, le fil 8 préalablement placé entre la première et la seconde arête de découpage, 20 et 22, peut être sectionné par cisaillement.

La pince coupante comporte un organe 24 de blocage du fil, monté pivotant sur l'axe 14. Comme on le voit mieux sur les figures 2 et 3, cet organe 24 présente une face intérieure 26 et une face extérieure 28. Conformément à la convention précédemment explicitée, la face intérieure 26 est celle qui est montée adjacente à la face latérale extérieure 30 du premier levier 10, cette face latérale extérieure étant celle qui est opposée au second levier 12.

L'organe 24 de blocage du fil présente une encoche 32, particulièrement visible sur les figures 1, 4 et 5. Cette encoche s'étend à partir de la périphérie de l'organe 24 vers sa partie centrale. Sa dimension est adaptée à recevoir le fil à sectionner. Concrètement, dans le cas d'une sonde de prélèvement microbiologique protégé, la tige de la brosse de prélèvement, qui constitue le fil à sectionner, est relativement fine, de sorte que l'encoche peut présenter une largeur de l'ordre de 1,3 mm.

Comme l'indiquent notamment les figures 1 et 2, en vue du découpage, l'organe 24 de blocage du fil est susceptible de venir par pivotement dans une position où le fil 8 peut être inséré dans l'encoche 32 et entre les première et seconde arêtes 20, 22 de découpage. Ce fil rigide 8 présente alors une partie extrême à couper 34 qui s'étend vers l'extérieur du premier levier 10, au-delà de la face extérieure 28 de l'organe de blocage 24. Dans le cas d'une sonde de prélèvement microbiologique, la brosse, non représentée, est fixée sur cette partie extrême à couper.

La figure 2 représente la position de l'organe 24 de blocage du fil et des leviers 10, 12 juste avant le découpage, tandis que la figure 3 représente la position de ces éléments juste après le découpage. On voit que, lors du découpage du fil, l'organe 24 de blocage du fil est susceptible d'être sollicité de manière à ramener une première face 36 de l'encoche 32, placée en vis-à-vis de la première arête de découpage 20, vers cette dernière. Cette première face active de l'encoche sera appelée dans la suite face active de l'organe de blocage. Ainsi, I'extrémité de la partie extrême à couper 34 du fil 8 partiellement engagée dans l'encoche 32 est maintenue bloquée entre la face active 36 de l'organe de blocage 24 et la première arête de découpage 20. Le fil est également maintenu bloqué même après le découpage, lorsque la seconde arête de découpage 22 a dépassé la première arête de découpage 20. Pour dégager la partie extrême coupée du fil, l'organe 24 de blocage du fil devra être sollicité en sens inverse de celui précédemment décrit.

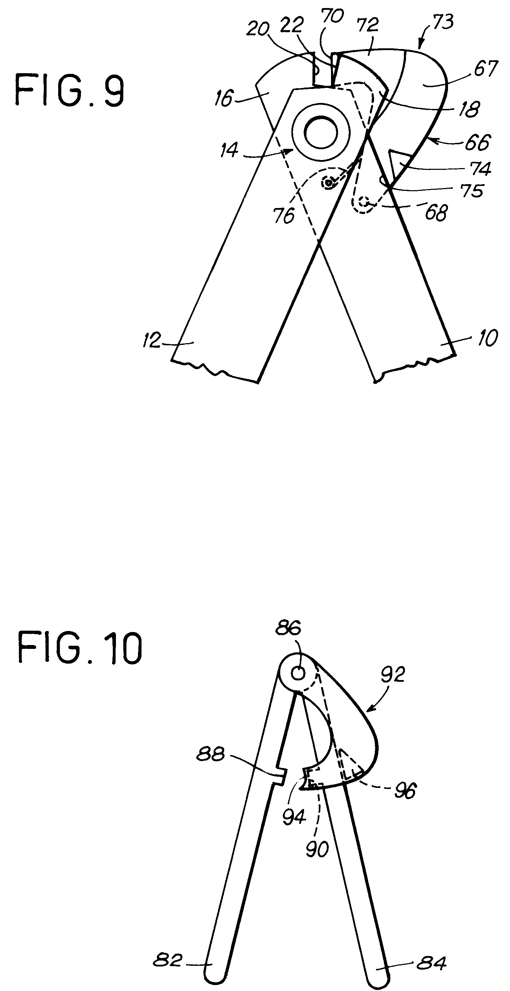

La figure 9 montre une autre variante de la pince coupante qui comporte un organe de blocage 66 monté pivotant sur un axe 68 solidaire du premier levier 10. L'axe 68 est parallèle à l'axe 14 de pivotement des leviers. L'organe de blocage 66 présente une face intérieure 67 et une face extérieure. La face intérieure 67 est montée adjacente au levier dont l'axe 68 de pivotement de l'organe de blocage est solidaire, c'est-à-dire, dans le cas représenté, adjacente au premier levier 10. L'organe de blocage 66 présente une face active 70 située sur sa périphérie et placée en vis-à-vis de la première arête de découpage 20.

En vue du découpage, l'organe 66 de blocage du fil 8 est susceptible, par pivotement autour de son axe 68, de venir dans une position où le fil 8 peut être inséré à la fois entre sa face active 70 et la première et la seconde arête de découpage. Dans cette position, le fil 8 présente une partie extrême à couper 34 qui s'étend vers l'extérieur du premier levier 10, au-delà de la face extérieure de l'organe de blocage 66.

Dans l'une ou l'autre de ces variantes, la pince comporte un contact de frottement apte à entraîner l'organe de blocage en pivotement avec le second levier lors du rapprochement des première et seconde arêtes de découpage.

Deux modes de réalisation de ce contact de frottement sont décrits dans la suite.

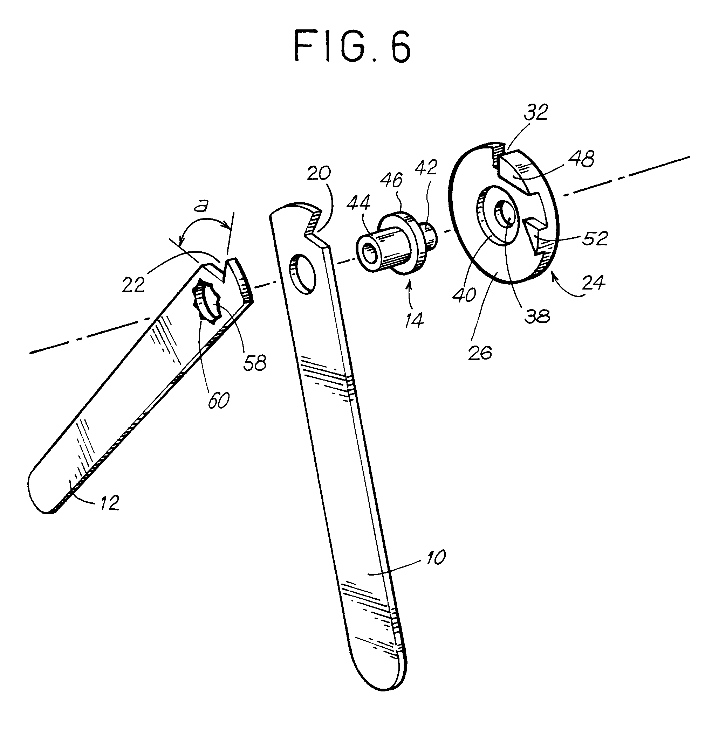

Par référence à la figure 6, on constate que l'organe de blocage 24 présente avantageusement un alésage étagé avec un alésage plus petit 38 et un alésage plus grand 40.

L'axe 14 comporte un épaulement annulaire de butée 46 qui le sépare en deux parties d'extrémités 42 et 44. Lorsque la pince est montée, cet épaulement annulaire de butée 46 sert d'un côté de butée axiale à l'organe 24 de blocage du fil, dont l'alésage étagé reçoit une première partie d'extrémité 42 de l'axe 14 et de l'autre côté, de butée axiale au premier levier 10.

Le diamètre de l'alésage le plus grand 40 de l'organe de blocage 24 est sensiblement égal au diamètre de l'épaulement 46. De plus, la profondeur de cet alésage 40 et l'épaisseur de l'épaulement 46 sont adaptées l'une à l'autre.

Ainsi, l'organe de blocage 24 étant placé sur la première partie d'extrémité 42 de l'axe 14 avec l'alésage le plus grand 40 adjacent à l'épaulement annulaire 46, cet alésage 40 coiffe l'épaulement 46 sur une épaisseur prédéterminée. Dans la mesure où les deux leviers 10 et 12 sont montés juxtaposés et sur la seconde partie d'extrémité 44 de l'axe 14, qui constitue leur axe d'articulation, le premier levier 10 et l'organe de blocage 24 peuvent pivoter indépendamment l'un de l'autre, au moins jusqu'à ce que la face active 36 de l'organe de blocage et la première arête de découpage 20 viennent, de part et d'autre du fil 8, en butée contre ce dernier.

Le premier levier 10 est monté en pivotement libre autour de l'axe 14 et le second levier 12 est solidaire en pivotement de cet axe. Au moins une face de l'alésage le plus grand 40 de l'organe de blocage 24 coopère par frottement avec l'épaulement annulaire de butée 46. Ce frottement est tel que lorsque, lors du découpage, le premier levier 10 est actionné en pivotement pour ramener la première arête de découpage 20 vers la seconde arête de découpage 22, l'organe de blocage 24 est entraîné vers la première arête de découpage 20, par l'intermédiaire de l'axe 14 et du second levier 12. Cet entraînement se poursuit jusqu'à ce que la face active 36 vienne au contact du fil rigide 8 à sectionner et le repousse en position bloquée contre la première arête de découpage 20. Le mouvement de pivotement se continuant, l'organe de blocage 24 reste fixe par rapport au premier levier 10, c'est-à-dire la partie coupée 34 du fil 8 reste bloquée.

Une fois le fil sectionné, il suffira, pour dégager la partie coupée du fil, de faire pivoter les leviers 10 et 12 vers la position d'ouverture de la pince en faisant s'écarter les arêtes de découpage. Lors de ce pivotement, l'organe de blocage 24 sera à nouveau entraîné par frottement, cette fois en sens inverse, de telle sorte que la face active 36 s'éloigne de la première arête de découpage 20 en relâchant la partie extrême coupée du fil.

Comme on le voit mieux sur les figures 2 et 3, I'extrémité de l'axe 14 proche de l'organe de blocage 24 est avantageusement refoulée sur elle-même à la façon d'un rivet, éventuellement jusqu'à repousser cet organe contre l'épaulement annulaire de butée 46, de manière à renforcer le frottement contre l'épaulement annulaire de butée ou contre la face intérieure du second levier.

Ainsi le contact par frottement entre l'épaulement 46 de l'axe 14 et l'alésage 40 de l'organe de blocage 24 peut être réglé.

De manière alternative ou complémentaire, comme particulièrement visible sur les figures 6 et 9, l'organe de blocage 24 ou 66 comporte une saillie 48 ou 72 formée le long de sa face active 36 ou 70 sur sa face intérieure 26 ou 67. Ainsi, au niveau de cette saillie 48, comme on le voit mieux sur les figures 2 et 3, la face intérieure de l'organe de blocage 24 ou 66 affleure la face intérieure 50 du bras court 18 du second levier 12 tournée vers le bras court 16 du premier levier 10. La zone d'affleurement se situe au moins au niveau de la première arête de découpage 20.

La partie extrême coupée 34 du fil 8 est donc susceptible d'être retenue par l'action concomitante de la face active 36 ou 70 de l'organe de blocage 24 pourvue de la saillie 48 ou 72 et de la première arête de découpage 20 du bras court 16 du premier levier 10.

Un contact par frottement jouant le même rôle que celui précédemment décrit peut être établi entre la saillie 48 et le bras court 18 du second levier 12. A cet effet, la face intérieure 26 de l'organe de blocage 24 présente une surépaisseur au niveau de la saillie 48. Le contact de frottement entre la saillie 48 et la face intérieure 50 du bras court 18 du second levier 12 peut donc être établi de telle sorte que lorsque, lors du découpage, la première arête de découpage 20 est ramenée vers la seconde arête de découpage 22, l'organe de blocage 24 est entraîné vers la première arête de découpage 20, par l'intermédiaire du levier 12. Ce mouvement d'entraînement a lieu jusqu'à ce que la face active 36 de l'organe de blocage 24 vienne au contact du fil 8 et le repousse en position bloquée contre la première arête de découpage 20. Comme décrit précédemment, cette position bloquée est maintenue lors du découpage et, dans l'exemple des figures 1 à 6, peut être relâchée par une simple réouverture des leviers 10 et 12 qui rétablit le contact de frottement entraînant, cette fois, l'organe de blocage 24 en sens inverse.

La saillie 72 de l'organe de blocage 66 peut être mise à contribution de la même manière, pour coopérer par frottement avec la face intérieure du bras court du second levier 12.

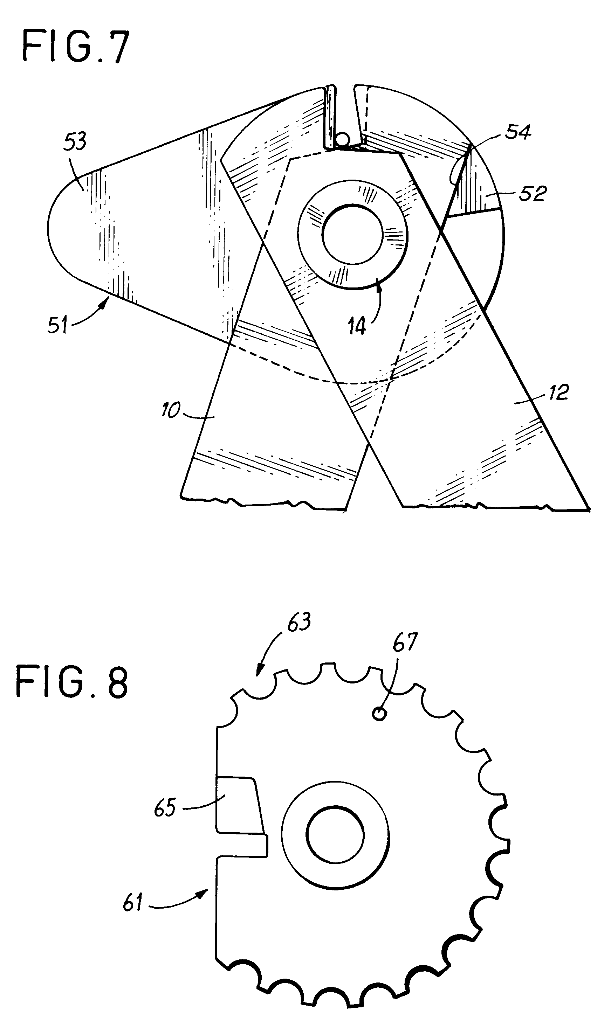

D'autres modes de réalisation de l'organe de blocage sont représentés aux figures 7 et 8. Cet organe peut prendre la forme d'un levier 51 ou d'une molette 61 et est susceptible d'être actionné manuellement en pivotement autour de l'axe 14, par exemple à l'aide du pouce.

Le levier 51 et la molette 61 comportent une zone de manipulation qui, dans le cas du levier, est constitué par un prolongement 53 et, dans le cas de la molette, est constituée par une denture 63 de la périphérie externe. Le contact de frottement précédemment décrit peut être mis en oeuvre, par exemple par l'intermédiaire de l'axe 14 ou d'une saillie 65, pour entraîner l'organe de blocage en pivotement jusqu'à une position de blocage du fil à couper.

Ensuite, pour relâcher la partie extrême coupée du fil, on sollicite l'organe de blocage manuellement pour éloigner sa face active de la première arête de découpage. Une telle manipulation permet de manoeuvrer la pince d'une seule main lors du relâchement de la partie extrême coupée du fil, sans devoir écarter les leviers.

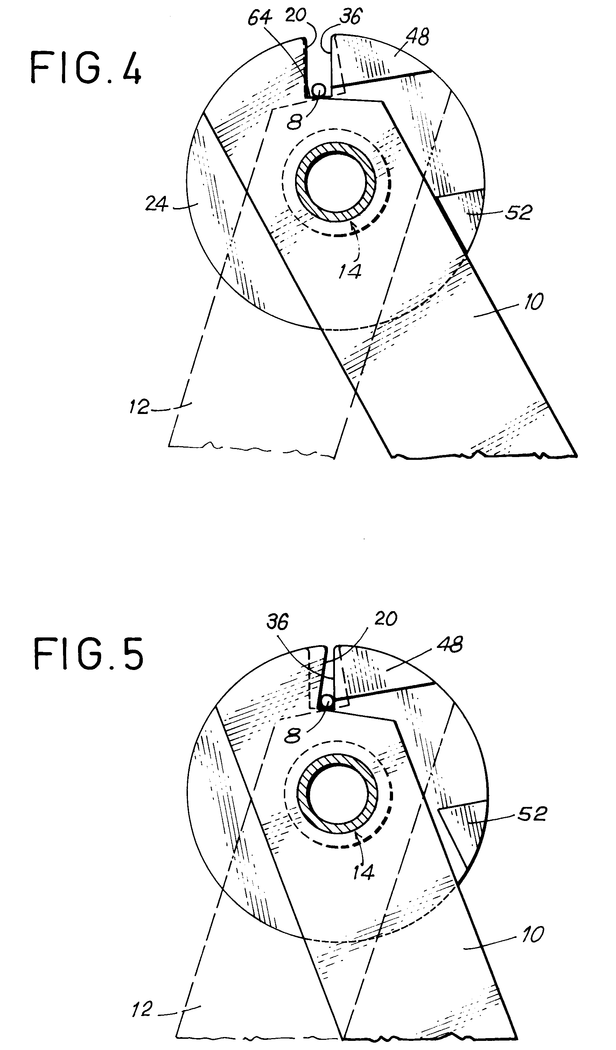

Comme le montrent clairement les figures 4 et 5, l'organe de blocage 24 suivant l'un des modes de réalisation évoqués comporte avantageusement une butée 52. Cette butée fait saillie à partir de la face intérieure 26 de l'organe 24, sensiblement parallèlement à l'axe 14 des leviers et vers le premier levier 10. Elle est située près de la périphérie de l'organe de blocage, écartée de la face active 36 et coopère avec une face de butée 54 du premier levier 10. Les figures 4 et 5 représentent cette butée 52 du côté de la face active 36 de l'organe de blocage et la face de butée 54 sur le bras long du levier 10. Conformément à la figure 7, une telle butée placée du côté opposé à la première face 36 de l'encoche 32 coopérant avec une face de butée située sur le bras court du levier 10 a le même effet. L'important est que la butée 52 et la face de butée 54 soient placées de telle sorte que, lorsqu'elles coopèrent en position d'ouverture de la pince, la face active 36 de l'organe de blocage soit positionnée entre la première et la seconde arête de découpage 20 et 22, et suffisamment écartée de la première arête 20 pour permettre l'insertion du fil 8 à sectionner.

Le cas échéant, la saillie 48 et la butée 52 peuvent être réalisées en continuité l'une de l'autre, dans un même renflement de la face interne de l'organe de blocage.

La butée 52 équipe également l'organe de blocage 51 de la figure 7. Sur la molette 61 de la figure 8, elle est réalisée sous la forme d'un téton 67.

Dans la variante de la figure 9, le contact de frottement est réalisé à l'aide de la saillie 72.

Si les surfaces en contact de la face interne du bras court de second levier d'une part, et de la face interne de la saillie 72 d'autre part sont suffisantes, ce contact de frottement sera suffisant à lui seul pour entraîner l'organe de blocage 66 vers la position de blocage. Dans ce cas, l'organe de blocage sera entraîné vers sa position de relâchement par action manuelle de l'utilisateur dans la zone 73. Pour faciliter cette manipulation et la rendre automatique, il peut être utile de prévoir un ressort de rappel 76 qui tend à écarter la face active de l'organe de blocage de la première arête de découpage.

La saillie 72 peut alors être conçue pour échapper au frottement du levier 12 si l'on continue de rapprocher les leviers 10 et 12 après le découpage du fil. Dans ce cas, l'organe de blocage se trouvera automatiquement libéré du contact de frottement après la coupe et s'écartera sous l'effet de la sollicitation du ressort 76, libérant ainsi la partie extrême coupée du fil.

Une butée de retenue 74 coopérant avec une face de butée 75 du premier levier 10 peut être prévue pour limiter l'écartement de la première arête et de la face active 70 de l'organe de blocage 66.

Selon l'un quelconque des modes de réalisation évoqués, le premier levier 10 est avantageusement monté en pivotement libre autour de l'axe 14 et le second levier 12 est avantageusement solidaire en pivotement de cet axe.

A cet effet, l'extrémité 56 de l'axe 14 proche du second levier 12 peut être refoulée sur elle-même à la façon d'un rivet de manière à coopérer, au moins par frottement, avec la surface de la face extérieure du second levier 12 adjacente à cette extrémité 56.

Comme l'indique la figure 6, pour améliorer la solidarisation en rotation, l'ouverture 58 du second levier 12 destinée à recevoir l'axe 14 peut comporter en bordure des rainures radiales 60. Ces rainures sont évidemment formées sur la face extérieure de ce levier 12, c'est-à-dire sur la face adjacente à l'extrémité refoulée 56 de l'axe 14. L'extrémité refoulée 56 de l'axe 14 est susceptible de s'ancrer dans ces rainures 60.

Les leviers 10 et 12, qui sont de préférence identiques, comportent avantageusement une découpe en "V" formée vers l'extrémité de leurs bras courts, 16 et 18. Une face sensiblement longitudinale de cette découpe en "V" forme l'arête de découpage 20 ou 22. L'autre face, 62, est sensiblement transversale.

A cet effet, et afin de s'assurer que la pince coupante soit manoeuvrable par une seule main, la première face de cette découpe qui constitue l'arête de découpage est avantageusement sensiblement parallèle à la face active 36 de l'organe de blocage 24 ou 66 et forme par exemple un angle de 25° avec l'axe longitudinal du levier considéré. Alors, lorsque la seconde face 62 forme avec la première face un angle a d'environ 90°, la condition d'adaptation précitée est remplie. Ainsi, l'angle entre les deux faces de cette découpe est tel que les deuxièmes faces de découpage en "V" des deux leviers restent sensiblement de même niveau, au moins sur une partie de la course de pivotement des leviers et tel que les deux arêtes de découpage soient en situation de cisaillement lorsque l'angle entre les leviers est d'environ 30°, de sorte que la pince coupante est manoeuvrable par une seule main et avec une force suffisante.

Selon le mode de réalisation présenté à la figure 10, la pince coupante comporte un premier levier 82 et un second levier 84. Ces deux leviers sont réunis, à une première extrémité, par un axe 86. Ils présentent une première et une seconde arêtes de découpage, 88 et 90, placées entre la première extrémité et une seconde extrémité opposée à l'axe 86. Ces deux arêtes de découpage 88 et 90 sont placées en vis-à-vis. Les premier et second leviers sont destinés à pivoter l'un par rapport à l'autre en rapprochant les arêtes de découpage l'une de l'autre, de manière à sectionner le fil préalablement placé entre ces arêtes.

Selon l'invention, cette pince comporte en outre un organe 92 de blocage du fil. Cet organe de blocage est monté pivotant sur l'axe 86 et présente une face intérieure et une face extérieure selon la convention précédemment explicitée. Il présente en outre une face active 94 située sur sa périphérie et placée en vis-à-vis de la première arête de découpage 88. Comme pour les autres modes de réalisation, en vue du découpage, l'organe de blocage 92 est susceptible de venir par pivotement dans une position où le fil peut être inséré à la fois entre sa face active et la première arête de découpage et entre les deux arêtes de découpage. La partie extrême à couper du fil s'étend alors au-delà de l'organe de blocage 92.

Lors du découpage, cet organe 92 peut être sollicité par un contact de frottement, de manière à ramener sa face active vers la première arête de découpage de façon à bloquer la partie extrême coupée du fil.

L'organe de blocage 92 peut également comporter une saillie jouant le même rôle que la saillie 48 précédemment évoquée.

Le montage de l'axe peut aussi être réalisé en refoulant l'une de ses extrémités ou les deux contre les éléments adjacents.

Les contacts de frottement entre l'axe et l'organe de blocage ou entre ce dernier et le levier adjacent peuvent être réalisés par les moyens précédemment évoqués.

Une butée 96 peut être prévue de manière que l'ouverture de la pince entraîne l'organe de blocage 92 en écartant sa face active 94 de la première arête de découpage 88. Cette butée 96 est formée sur la face intérieure de l'organe de blocage 92 et s'étend selon l'axe 86.

Bien entendu, diverses modifications peuvent être apportées par l'homme de l'art à la pince coupante qui vient d'être décrite, sans sortir du cadre de l'invention.

## Revendications

**1.** Pince coupante stérile destinée à sectionner un fil (8) plus ou moins rigide, comprenant un premier levier (10) et un second levier (12) réunis par un axe (14) qui sépare chaque levier en un bras long et un bras court, les bras courts (16, 18) des premier et second leviers présentant, près de leurs extrémités, une première et une seconde arêtes de découpage (20, 22) placées en vis-à-vis, lesdits premier et second leviers (10, 12) étant destinés à pivoter l'un par rapport à l'autre en rapprochant les première et seconde arêtes de découpage (20, 22) l'une de l'autre, de manière à sectionner le fil (8) préalablement placé entre lesdites première et seconde arêtes de découpage (20, 22), la pince comportant, en outre, un organe (24) de blocage du fil (8), monté pivotant sur l'axe (14) et présentant une face intérieure (26) et une face extérieure (28), ladite face intérieure étant montée adjacente à la face latérale extérieure (30) de l'un des leviers, dit premier levier (10), opposée à l'autre levier, dit second levier (12), cet organe (24) de blocage du fil présentant une encoche (32) s'étendant, à partir de la périphérie dudit organe, vers sa partie centrale et de dimension adaptée à recevoir le fil (8) à sectionner, en vue du découpage, l'organe (24) de blocage du fil étant susceptible de venir par pivotement dans une position où le fil (8) peut être inséré dans l'encoche (32) et entre lesdites première et seconde arêtes de découpage (20, 22), ledit fil (8) présentant alors une partie extrême à couper (34) s'étendant vers l'extérieur du premier levier (10), au-delà de la face extérieure (28) de l'organe de blocage (24), caractérisée :
- en ce qu'elle comporte un contact de frottement (40, 46 ; 48) apte lors du rapprochement des première et seconde arêtes de découpage (20, 22), à entraîner l'organe de blocage (24) en pivotement avec le second levier (12), ledit organe de blocage (24) étant ainsi susceptible d'être sollicité par frottement lors du découpage, de manière à ramener une face active (36) constituée par une face de l'encoche (32), placée en vis-à-vis de la première arête de découpage (20), vers ladite première arête de découpage (20), de telle sorte que l'extrémité de la partie extrême coupée (34) du fil (8) partiellement engagée dans l'encoche (32) soit maintenue bloquée entre ladite face active (36) et ladite première arête de découpage (20).

**2.** Pince coupante stérile destinée à sectionner un fil (8) plus ou moins rigide, comprenant un premier levier (82) et un second levier (84) réunis, à une première extrémité, par un axe (86), lesdits premier et second leviers (82, 84) présentant, entre ladite première extrémité et une seconde extrémité opposée à l'axe (86), une première et une seconde arête de découpage (88, 90) placées en vis-à-vis, les premier et second leviers (82, 84) étant destinés à pivoter l'un par rapport à l'autre en rapprochant les première et seconde arêtes de découpage (88, 90) l'une de l'autre, de manière à sectionner le fil (8) préalablement placé entre lesdites première et seconde arêtes de découpage (88, 90), la pince comportant, en outre, un organe (92) de blocage du fil (8), monté pivotant sur l'axe (86) et présentant une face intérieure et une face extérieure, cet organe (92) de blocage du fil (8) présentant une face active (94) située sur la périphérie dudit organe de blocage (92), en vis-à-vis de la première arête de découpage (88), en vue du découpage, l'organe (92) de blocage du fil (8) étant susceptible, par pivotement autour de son axe (86), de venir dans une position où le fil (8) peut être inséré entre ladite face active (94) et la première arête de découpage (88) et entre lesdites première et seconde arêtes de découpage (88, 90), ledit fil présentant alors une partie extrême à couper s'étendant au-delà dudit organe de blocage (92), caractérisé :
- en ce qu'elle comporte un contact de frottement (40, 46 ; 48) apte, lors du rapprochement des première et seconde arêtes de découpage (88, 90), à entraîner l'organe de blocage (92) en pivotement avec le second levier (84), ledit organe de blocage (92) étant ainsi susceptible d'être sollicité par frottement lors du découpage de manière à ramener ladite face active (94) vers ladite première arête de découpage (88), de telle sorte que l'extrémité de la partie extrême à couper partiellement engagée entre la face active (94) et la première arête de découpage (88) soit maintenue bloquée entre ladite face active (94) et ladite première arête de découpage (88).

**3.** Pince coupante stérile destinée à sectionner un fil (8) plus ou moins rigide, comprenant un premier levier (10) et un second levier (12) réunis par un axe (14) qui sépare chaque levier en un bras long et un bras court, les bras courts (16, 18) des premier et second leviers présentant, près de leurs extrémités, une première et une seconde arêtes de découpage (20, 22) placées en vis-à-vis, lesdits premier et second leviers (10, 12) étant destinés à pivoter l'un par rapport à l'autre en rapprochant les première et seconde arêtes de découpage (20, 22) l'une de l'autre, de manière à sectionner le fil (8) préalablement placé entre lesdites première et seconde arêtes de découpage (20, 22), la pince comportant un organe (66) de blocage du fil (8) présentant une face active (70) située, sur la périphérie dudit organe de blocage, en vis-à-vis de la première arête de découpage (20), caractérisée :
- en ce que l'organe de blocage (66) est monté pivotant sur un axe (68) solidaire de l'un des leviers (10, 12), décalé par rapport à l'axe (14) de pivotement desdits leviers (10, 12), et parallèle audit axe (14), ledit organe de blocage présentant une face intérieure (67) et une face extérieure, ladite face intérieure (67) étant montée adjacente à celui des deux leviers (10, 12) dont l'axe (68) de pivotement de l'organe de blocage (66) est solidaire,
- en ce que, en vue du découpage, l'organe (66) de blocage du fil (8) est susceptible, par pivotement autour de son axe (68), de venir dans une position où le fil (8) peut être inséré entre ladite face active (70) et la première arête de découpage (20) et entre lesdites première et seconde arêtes de découpage (20, 22), ledit fil (8) présentant alors une partie extrême à couper (34) s'étendant vers l'extérieur du premier levier (10), au-delà de la face extérieure dudit organe de blocage (66), et
- et en ce qu'elle comporte un contact de frottement (72) apte, lors du rapprochement des première et seconde arêtes de découpage (20, 22), à entraîner l'organe de blocage (66) en pivotement avec le second levier (12), ledit organe de blocage (66) étant ainsi susceptible d'être sollicité par frottement lors du découpage de manière à ramener ladite face active (70) vers ladite première arête de découpage (20), de telle sorte que l'extrémité (34) de la partie extrême coupée du fil (8) partiellement engagée entre la face active (70) et la première arête de découpage (20) soit maintenue bloquée entre ladite face active (70) et ladite première arête de découpage (20).

**4.** Pince coupante selon l'une quelconque des revendications 1 à 3, caractérisée en ce que le contact de frottement (40, 46 ; 48 ; 72) est apte, lors de l'écartement des arêtes de découpage (20, 22 ; 88, 90), à entraîner l'organe de blocage (24 ; 66; 92) en pivotement avec le second levier (12 ; 84), ledit organe de blocage (24 ; 66 ; 92) étant ainsi susceptible d'être sollicité par frottement lors de l'ouverture de la pince, de manière à éloigner la face active (36 ; 70 ; 94) de la première arête de découpage (20 ; 88) en relâchant la partie extrême coupée du fil.

**5.** Pince coupante selon l'une quelconque des revendications 1 à 4, caractérisée :
- en ce que l'organe (24, 66, 92) de blocage du fil (8) comporte une saillie (48, 72) formée le long de la face active (36, 70, 94), sur la face intérieure (26, 67) dudit organe (24, 66, 92) et s'étendant parallèlement à l'axe (14, 86), de pivotement des premier et second leviers (10, 12 ; 82, 84), au minimum de telle sorte que, au niveau de ladite saillie (48, 72), la face intérieure (26, 67) dudit organe (24, 66, 92) affleure la face intérieure (50) du second levier (12, 84) tournée vers le premier levier (10 ; 82), au moins au niveau de la première arête de découpage (20, 88), et
- en ce que la partie extrême coupée (34) du fil (8) est susceptible d'être retenue par l'action concomitante de la face active (36, 70, 94) de l'organe de blocage (24, 66, 92) pourvue de la saillie (48, 72) et de la première arête de découpage (20, 88) du premier levier (10, 82).

**6.** Pince coupante selon la revendication 5, caractérisée en ce que, au niveau de la saillie (48, 72), la face intérieure (26, 67) de l'organe de blocage (24, 66, 92) présente une surépaisseur de manière à coopérer par frottement avec la face intérieure (50) du second levier (12, 84) de telle sorte que, lors du découpage, la première arête de découpage (20, 88) étant ramenée vers la seconde arête de découpage (22, 90), ledit organe de blocage (24, 92) soit entraîné vers la première arête de découpage (20, 88) par l'intermédiaire du second levier (12, 84) jusqu'à ce que la face active (36, 94) dudit organe de blocage (24, 92) vienne au contact du fil (8) à sectionner et repousse ledit fil (8) en position bloquée entre ladite face active (36, 94) et ladite première arête de découpage (20, 88).

**7.** Pince coupante selon l'une quelconque des revendications 1, 2 et 4 à 6, caractérisée
- en ce que l'organe de blocage (24, 92) présente un alésage étagé (38, 40) destiné à recevoir l'axe (14, 86),
- en ce que l'axe (14, 86) comporte deux parties d'extrémité (42, 44) séparées par un épaulement annulaire de butée (46) servant de butée axiale d'un côté à l'organe de blocage (24, 84), et de l'autre côté au premier levier (10, 82), le diamètre de l'alésage le plus grand (40) de l'organe de blocage étant sensiblement égal au diamètre de l'épaulement annulaire de butée (46) et la profondeur dudit alésage le plus grand (40) étant adaptée de telle manière que, l'organe de blocage (24, 92) étant placé sur la première partie d'extrémité (42) de l'axe (14, 86) avec l'alésage le plus grand (40) adjacent à l'épaulement annulaire de butée (46), ledit alésage le plus grand (40) coiffe ledit épaulement annulaire de butée (46) sur une épaisseur prédéterminée, et
- en ce que la seconde partie d'extrémité (44) de l'axe (14) constitue l'axe d'articulation des premier et second leviers (10, 12 ; 82, 84) juxtaposés.

**8.** Pince coupante selon la revendication 7, caractérisée :
- en ce que le premier levier (10, 82) est monté en pivotement libre autour de l'axe (14, 86),
- en ce que le second levier (12, 84) est solidaire en pivotement dudit axe (14, 86), et
- et en ce qu'au moins une face de l'alésage le plus grand (40) de l'organe de blocage (24, 92) coopère par frottement avec l'épaulement annulaire de butée (46) de l'axe (14, 86) de telle sorte que lors du découpage, le premier levier (10, 82) étant actionné en pivotement pour ramener la première arête de découpage (20, 88) vers la seconde arête de découpage (22, 90), ledit organe de blocage (24, 92) soit entraîné, par l'intermédiaire de l'axe (14, 86) et du second levier (12, 84), vers la première arête de découpage (20, 88) jusqu'à ce que la face active (36, 94) de l'organe de blocage (24, 92) vienne au contact du fil (8) à sectionner et repousse ledit fil (8) en position bloquée entre ladite face active (36, 94) et ladite première arête de découpage (20, 88).

**9.** Pince coupante selon la revendication 8, caractérisée en ce que l'extrémité de l'axe (14, 86) proche de l'organe de blocage (24, 92) est refoulée sur elle-même à la façon d'un rivet, de manière à coopérer par frottement avec la face extérieure (28) de l'organe de blocage (24, 92) et à repousser celui-ci contre l'épaulement annulaire de butée (46) en réglant le contact par frottement entre ledit épaulement annulaire de butée (46) et au moins la face de l'alésage le plus grand (40) de l'organe de blocage (24, 92).

**10.** Pince selon l'une quelconque des revendications 1 à 9, caractérisée en ce que l'organe (24, 66, 92) de blocage du fil (8) présente une butée (52, 74, 96) faisant saillie à partir de sa face intérieure (26, 67), sensiblement parallèlement à l'axe (14, 86) de pivotement des leviers (10, 12, 82, 84) et vers le premier levier (10, 82), ladite butée (52, 74, 96) étant située près de la périphérie de l'organe de blocage (24, 66, 92) et écartée de la face active (36, 70, 94) dudit organe (24, 66, 92) de telle sorte que, lorsque la face active de cette butée (52, 74, 96) coopère avec une face de butée (54, 75) du levier adjacent (10, 84) en position d'ouverture de la pince, la face active (36, 70, 94) de l'organe de blocage (24, 66, 92) soit positionnée entre la première et la seconde arête de découpage (20, 22, 88, 90) et suffisamment écartée de ladite première arête de découpage (20, 88) pour permettre l'insertion du fil (8).

**11.** Pince coupante selon l'une quelconque des revendications 1 à 10, caractérisée en ce que le premier levier (10, 82) est monté en pivotement libre autour de l'axe (14, 86) et en ce que le second levier (12, 84) est solidaire en pivotement dudit axe (14, 86).

**12.** Pince selon la revendication 11, caractérisée en ce que l'extrémité de l'axe (14, 86) proche du second levier (12, 84) est refoulée sur elle-même vers l'extérieur à la façon d'un rivet (56), de manière à coopérer, au moins par frottement, avec la surface du second levier (12, 84) adjacente à ladite extrémité de l'axe (14, 86).

**13.** Pince selon la revendication 12, caractérisée en ce que l'ouverture (58) du second levier (12, 84) destinée à recevoir l'axe (14, 86) comporte, en bordure, des rainures radiales (60) formées sur la face dudit levier (12, 84) adjacente à l'extrémité refoulée (56) dudit axe (14, 86), ladite extrémité refoulée (56) étant susceptible de s'ancrer dans lesdites rainures radiales (60).

**14.** Pince coupante selon l'une quelconque des revendications 1 à 13, caractérisée en ce que les premier et second leviers (10, 12) comportent une découpe en "V" dont une première face, sensiblement longitudinale, constitue les arêtes de découpage (20, 22) desdits leviers (10, 12) et dont la seconde face (62) est sensiblement transversale, les angles desdites première et seconde faces (20, 22, 62) de la découpe en "V" étant adaptés de telle sorte que l'angle entre les premier et second leviers (10, 12) en position de découpage est d'environ 30° et que les secondes faces (62) des découpes en "V" des leviers (10, 12) restent sensiblement de même niveau, au moins sur une partie du pivotement des premier et second leviers (10, 12) l'un par rapport à l'autre.

**15.-** Pince coupante selon l'une quelconque des revendications 1 à 3 et 5 à 14, caractérisée en ce que l'organe de blocage (61, 51, 66) comporte une zone de manipulation (53, 63, 73), permettant de le solliciter manuellement pour écarter la face active dudit organe de blocage (51, 61, 66) de la première arête de découpage (20).

**16.** Pince coupante selon l'une quelconque des revendications 3, 5, 6, 10, 14 et 15, caractérisée en ce qu'elle comporte un ressort de rappel (76) qui tend à écarter la face active (70) de l'organe de blocage (66) de la première arête de découpage (20).

**17.** Pince coupante selon les revendications 6 et 16, caractérisée en ce que la saillie (72) est conçue de manière à échapper au frottement du levier (12) lorsque l'on continue de rapprocher les leviers (10, 12) après le découpage, et en ce que le ressort de rappel (76) repousse alors l'organe de blocage (66) de manière à libérer la partie extrême coupée du fil.
